(19) Europäisches Patentamt
European Patent Office
Office européen des brevets

(11) **EP 4 394 028 A1**

(12) **EUROPEAN PATENT APPLICATION**
published in accordance with Art. 153(4) EPC

(43) Date of publication:
**03.07.2024 Bulletin 2024/27**

(21) Application number: **22861016.8**

(22) Date of filing: **20.07.2022**

(51) International Patent Classification (IPC):
*C12N 5/00* (2006.01)    *C12M 1/26* (2006.01)
*C12M 3/00* (2006.01)    *C12N 1/04* (2006.01)
*C12N 5/078* (2010.01)

(52) Cooperative Patent Classification (CPC):
**C12M 1/26; C12M 3/00; C12N 1/04; C12N 5/00; C12N 5/06**

(86) International application number:
**PCT/JP2022/028168**

(87) International publication number:
**WO 2023/026725 (02.03.2023 Gazette 2023/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.08.2021 JP 2021137171**

(71) Applicant: **Sekisui Medical Co., Ltd.**
**Tokyo 103-0027 (JP)**

(72) Inventors:
• **UCHIYAMA, Takaya**
  **Shunan-shi, Yamaguchi 746-0006 (JP)**
• **KOMAI, Kuniya**
  **Shunan-shi, Yamaguchi 746-0006 (JP)**
• **INOUE, Tomonori**
  **Shunan-shi, Yamaguchi 746-0006 (JP)**

(74) Representative: **Ter Meer Steinmeister & Partner Patentanwälte mbB**
**Nymphenburger Straße 4**
**80335 München (DE)**

(54) **STORAGE SOLUTION FOR CELL-CONTAINING SOLUTION AND STORAGE CONTAINER FOR CELL-CONTAINING SOLUTION**

(57)    Provided is a storage solution for a cell-containing solution capable of suppressing leakage of genomic DNA to the outside of cells when a mixed solution of a cell-containing solution and a storage solution is stored under refrigeration. A storage solution for a cell-containing solution according to the present invention contains a formaldehyde donor compound (A) and a compound (B) that is an inorganic salt, an organic acid, or an organic salt, an electrical conductivity of the storage solution being 3 ms/cm or more and 20 ms/cm or less.

**EP 4 394 028 A1**

## Description

### TECHNICAL FIELD

The present invention relates to a storage solution used for storing a cell-containing solution. The present invention also relates to a storage container for a cell-containing solution, the storage container for a cell-containing solution including the storage solution.

### BACKGROUND ART

Body fluids such as blood, plasma, serum, and urine include free DNA (cell free DNA, cfDNA). It is known that cfDNA is present in a body fluid of a person having a malignant tumor or a person suffering from an infectious disease at a higher concentration than that of a healthy person.

In recent years, a test using cfDNA as a specimen, and the like have been performed in regions such as cancer and genetic diseases. The test using cfDNA as a specimen has a smaller burden on a patient than a test performed by collecting the diseased tissue from the patient.

Maternal blood includes fetal free DNA (cell free fetal DNA, cffDNA). In a prenatal genetic test, a test using cffDNA as a specimen is performed.

It is difficult to perform the test using cfDNA as a specimen unless a specialized institution having a dedicated device such as a qPCR device or a next generation sequencer (NGS) performs such a test. Therefore, it is necessary to transport the cell-containing solution such as blood collected in a hospital or the like to a specialized institution, and a certain number of days are required from collection to analysis of the cell-containing solution. During this time, the cell-containing solution is stored in a predetermined container.

Patent Documents 1 and 2 below describe methods for stably recovering cfDNA by stabilizing cells, particularly white blood cells, present in whole blood.

Patent Document 3 below discloses a vitrified cryopreservation solution for animal cells, containing, as a cryoprotective material, at least one water-soluble cellulose-based cryoprotective material selected from the group consisting of hydroxypropyl cellulose, hydroxypropyl methylcellulose, and hydroxyethyl methylcellulose. Patent Document 3 describes that the vitrified cryopreservation solution preferably contains a specific cell-membrane-permeable cryoprotective material or a specific cell-membrane-impermeable cryoprotective material.

### Related Art Documents

### Patent Documents

Patent Document 1: WO 2013/123030 A1
Patent Document 2: CN 107083382 A
Patent Document 3: JP 2011-111406 A

## SUMMARY OF THE INVENTION

### PROBLEMS TO BE SOLVED BY THE INVENTION

A test using cfDNA contained in a cell-containing solution such as blood as a specimen is performed. The cell-containing solution such as blood is collected in a storage container in which a storage solution has been stored, and then transported to a specialized institution having a qPCR device or a next generation sequencer. The transportation to the specialized institution may be performed in a frozen environment, but it is desirable to perform the transportation in a refrigerated environment from the viewpoint of suppressing the transportation cost and suppressing the trouble during the transportation.

However, in the conventional methods as described in Patent Documents 1 to 3, when the cell-containing solution is stored in a refrigerated environment, genomic DNA (gDNA) may leak from cells. For example, in a conventional storage container in which a storage solution has been stored, when a cell-containing solution is stored in a refrigerated environment, cells contained in the cell-containing solution may be destroyed or killed, and genomic DNA may leak from the cells.

In the test using cfDNA as a specimen, when genomic DNA leaks out of cells, the test result is greatly affected.

An object of the present invention is to provide a storage solution for a cell-containing solution capable of suppressing leakage of genomic DNA to the outside of cells when a mixed solution of a cell-containing solution and a storage solution is stored under refrigeration. Another object of the present invention is to provide a storage container for a cell-containing solution, the storage container including the storage solution for a cell-containing solution.

**MEANS FOR SOLVING THE PROBLEMS**

According to a broad aspect of the present invention, there is provided a storage solution for a cell-containing solution (hereinafter, abbreviated as "storage solution") containing a formaldehyde donor compound (A) and a compound (B) that is an inorganic salt, an organic acid, or an organic salt, an electrical conductivity of the storage solution being 3 ms/cm or more and 20 ms/cm or less.

In a specific aspect of the storage solution of the present invention, the storage solution contains a cell-membrane-permeable compound (C) having a molecular weight of 100 or less and being not frozen at 0°C.

In a specific aspect of the storage solution of the present invention, the cell-membrane-permeable compound (C) is a polyhydric alcohol.

In a specific aspect of the storage solution of the present invention, the cell-membrane-permeable compound (C) is ethylene glycol, propylene glycol, glycerin, dimethyl sulfoxide, acetamide, 1,3-propanediol, or butylene glycol.

In a specific aspect of the storage solution of the present invention, the storage solution contains a cell-membrane-impermeable compound (D) having a molecular weight of 300 or more.

In a specific aspect of the storage solution of the present invention, the molecular weight of the cell-membrane-impermeable compound (D) is 1000 or more.

In a specific aspect of the storage solution of the present invention, the cell-membrane-impermeable compound (D) is polyvinylpyrrolidone, polyethylene glycol, polyvinyl alcohol, a polysaccharide, a polysaccharide derivative, sugar alcohol, or ficoll.

In a specific aspect of the storage solution of the present invention, the formaldehyde donor compound (A) is DMDM hydantoin or 1-hydroxymethyl-5,5-dimethylhydantoin.

In a specific aspect of the storage solution of the present invention, the compound (B) is citric acid, a citrate, or sodium chloride.

In a specific aspect of the storage solution of the present invention, the cell-containing solution is blood.

According to a broad aspect of the present invention, there is provided a storage container for a cell-containing solution in which a predetermined amount of a cell-containing solution is collected, the storage container for a cell-containing solution, including: a container body; and a storage solution stored inside the container body, the storage solution being the above-described storage solution for a cell-containing solution.

In a specific aspect of the storage container for a cell-containing solution of the present invention, the storage container for a cell-containing solution includes the following configuration P:

Configuration P: when 7.4 g of sodium hypochlorite is mixed with 1 L of physiological saline to obtain a solution Pa, the solution Pa is collected in the storage container for a cell-containing solution in an amount equivalent to a predetermined amount of the cell-containing solution collected in the storage container for a cell-containing solution, and the solution Pa and the storage solution are mixed to obtain a mixed solution P, a content of formaldehyde in the mixed solution P is 100 mg/L or more and 400 mg/L or less.

In a specific aspect of the storage container for a cell-containing solution of the present invention, the storage container for a cell-containing solution includes the following configuration Q:

Configuration Q: when physiological saline is collected in the storage container for a cell-containing solution in an amount equivalent to a predetermined amount of the cell-containing solution collected in the storage container for a cell-containing solution, and the physiological saline and the storage solution are mixed to obtain a mixed solution Q, an osmotic pressure of the mixed solution Q is 300 mOsm/L or more and 1100 mOsm/L or less.

In a specific aspect of the storage container for a cell-containing solution of the present invention, the storage solution contains a cell-membrane-permeable compound (C) having a molecular weight of 100 or less and being not frozen at 0°C, and the storage container for a cell-containing solution includes the following configuration R:

Configuration R: when the predetermined amount of the cell-containing solution is collected in the storage container for a cell-containing solution, and the cell-containing solution and the storage solution are mixed to obtain a mixed solution R, a content of the cell-membrane-permeable compound (C) in the mixed solution R is 1 vol% or more and 5 vol% or less.

In a specific aspect of the storage container for a cell-containing solution of the present invention, the storage solution contains a cell-membrane-impermeable compound (D) having a molecular weight of 300 or more, and the storage container for a cell-containing solution includes the following configuration S:

Configuration S: when the predetermined amount of the cell-containing solution is collected in the storage container for a cell-containing solution, and the cell-containing solution and the storage solution are mixed to obtain a mixed solution S, a content of the cell-membrane-impermeable compound (D) in the mixed solution S is 0.5 µmol/L or

more and 5 μmol/L or less.

**EFFECT OF THE INVENTION**

A storage solution for a cell-containing solution according to the present invention contains a formaldehyde donor compound (A) and a compound (B) that is an inorganic salt, an organic acid, or an organic salt, an electrical conductivity of the storage solution being 3 ms/cm or more and 20 ms/cm or less. Since the storage solution for a cell-containing solution according to the present invention has the above configuration, it is possible to suppress leakage of genomic DNA to the outside of cells when a mixed solution of a cell-containing solution and a storage solution is stored under refrigeration.

**MODES FOR CARRYING OUT THE INVENTION**

Hereinafter, the present invention will be described in detail.

A storage solution for a cell-containing solution according to the present invention (hereinafter, abbreviated as "storage solution") contains a formaldehyde donor compound (A) and a compound (B) that is an inorganic salt, an organic acid, or an organic salt, an electrical conductivity of the storage solution being 3 ms/cm or more and 20 ms/cm or less.

Since the storage solution according to the present invention has the above configuration, it is possible to suppress leakage of genomic DNA to the outside of cells when a mixed solution of a cell-containing solution and a storage solution is stored under refrigeration. In the storage solution according to the present invention, for example, it is possible to suppress leakage of genomic DNA to the outside of cells even when a mixed solution of a cell-containing solution and a storage solution is stored at 4°C. In the present invention, it is possible to effectively suppress contamination of genomic DNA in cells into the cell-containing solution due to destruction or death of cells contained in the cell-containing solution during storage in a refrigerated environment. Therefore, in the present invention, the storage stability of cfDNA can be enhanced.

The cell-containing solution is a liquid containing cells. Examples of the cell-containing solution include body fluids, and specific examples thereof include blood such as whole blood, urine, and cerebrospinal fluid. The blood includes white blood cells and the like as cells.

From the viewpoint of more effectively exhibiting the effect of the present invention, the cell-containing solution is preferably blood. The storage solution is preferably a storage solution for blood. When the cell-containing solution is blood, in the present invention, hemolysis during refrigerated storage can also be effectively suppressed.

(Storage solution for cell-containing solution)

The storage solution is a liquid at 25°C.

From the viewpoint of exhibiting the effect of the present invention, the electrical conductivity of the storage solution is 3 ms/cm or more and 20 ms/cm or less.

The electrical conductivity of the storage solution is preferably 3.5 ms/cm or more, more preferably 4 ms/cm or more, and further preferably 4.5 ms/cm or more, and preferably 18 ms/cm or less, more preferably 14 ms/cm or less, further preferably 10 ms/cm or less, and particularly preferably 7 ms/cm or less. When the electrical conductivity is the lower limit or more and the upper limit or less, the effect of the present invention can be more effectively exhibited. When the electrical conductivity is the lower limit or more and the upper limit or less, hemolysis can be more effectively suppressed when the cell-containing solution is blood.

The electrical conductivity of the storage solution is measured at 25.0°C using a cell having a cell constant of $0.949 \times 0.1$ cm$^{-1}$ and an electrical conductivity meter. Specifically, the electrical conductivity can be measured by immersing an electrode in 15 mL of the storage solution. As the electrical conductivity meter, for example, "DS-71" manufactured by HORIBA Scientific can be used.

<Formaldehyde donor compound (A)>

The storage solution contains a formaldehyde donor compound (in the present specification, referred to as "formaldehyde donor compound (A)"). The formaldehyde donor compound (A) is a compound capable of releasing formaldehyde. By using the formaldehyde donor compound (A), when the cell-containing solution and the storage solution are mixed, the membrane protein of the cell is crosslinked by the action of formaldehyde released from the formaldehyde donor compound (A), and the stability of the cell can be enhanced. In a conventional storage solution containing a formaldehyde donor compound, the fragment length of cfDNA contained in the cell-containing solution is likely to change during storage in a refrigerated environment. On the other hand, in the present invention, even when the storage solution contains a formaldehyde donor compound, the fragment length change of cfDNA can be made less likely to occur. Only one kind of the formaldehyde donor compound (A) may be used, or two or more kinds thereof may be used in combination.

Examples of the formaldehyde donor compound (A) include a hydantoin compound, imidazolidinylurea, diazolidinylurea, hexamethylenetetramine, N,N''-methylenebis-[N'-(3-hydroxymethyl-2,5-dioxo-4-imidazolidinyl)urea], a tertiary amine compound, a secondary amine compound, and a primary amine compound.

Examples of the hydantoin compound include DMDM hydantoin, 1-hydroxymethyl-5,5-dimethylhydantoin, 1,3-dimethylol-5,5-hydantoin, and 1,3-dichloro-5,5-dimethylhydantoin.

From the viewpoint of more effectively exhibiting the effect of the present invention, the formaldehyde donor compound (A) is preferably a hydantoin compound, and more preferably DMDM hydantoin or 1-hydroxymethyl-5,5-dimethylhydantoin.

The content of the formaldehyde donor compound (A) in the storage solution is not particularly limited. The content of the formaldehyde donor compound (A) in the storage solution can be appropriately changed, for example, so that the content of formaldehyde satisfies the range described below in the mixed solution P described below. The content of the formaldehyde donor compound (A) in the storage solution can be appropriately changed according to the mixing ratio of the cell-containing solution and the storage solution, the type of the formaldehyde donor compound (A), and the like.

The content of the formaldehyde donor compound (A) in the storage solution is preferably 0.1 w/v% or more, more preferably 0.5 w/v% or more, and further preferably 1 w/v% or more, and preferably 5.0 w/v% or less, more preferably 4.5 w/v% or less, and further preferably 4.0 w/v% or less. When the content of the formaldehyde donor compound (A) is the lower limit or more and the upper limit or less, formaldehyde is favorably released from the formaldehyde donor compound (A), and as a result, the stability of cells can be enhanced and the decomposition of cfDNA can be suppressed.

<Compound (B) that is inorganic salt, organic acid, or organic salt>

The storage solution contains a compound that is an inorganic salt, an organic acid, or an organic salt (in the present specification, referred to as "compound (B)"). By using the compound (B), the ionic strength of the storage solution can be favorably adjusted, and the electrical conductivity of the storage solution can be adjusted to an appropriate range. The compound (B) may be an inorganic salt, an organic acid, an organic salt, or a mixture thereof. The compound (B) is at least one compound selected from the group consisting of an inorganic salt, an organic acid, and an organic salt. Only one kind of the compound (B) may be used, or two or more kinds thereof may be used in combination.

Examples of the inorganic salt include sodium chloride, calcium chloride, potassium chloride, magnesium chloride, a phosphate, a carbonate, and a borate. Examples of the phosphate include sodium phosphate, sodium dihydrogen phosphate, disodium hydrogen phosphate, trisodium phosphate, potassium dihydrogen phosphate, dipotassium hydrogen phosphate, and dipotassium phosphate. Examples of the carbonate include sodium carbonate, sodium hydrogen carbonate, potassium carbonate, potassium hydrogen carbonate, and ammonium carbonate. Examples of the borate include sodium borate. Only one kind of the inorganic salt may be used, or two or more kinds thereof may be used in combination.

Examples of the organic acid include citric acid, succinic acid, fumaric acid, malic acid, adipic acid, tartaric acid, benzoic acid, pyrrolidone carboxylic acid, and salicylic acid. Only one kind of the organic acid may be used, or two or more kinds thereof may be used in combination.

Examples of the organic salt include a citrate, a succinate, and an acetate. Examples of the citrate include sodium dihydrogen citrate, sodium citrate, disodium citrate, and trisodium citrate. Examples of the succinate include disodium succinate. Examples of the acetate include calcium acetate and sodium acetate. Only one kind of the organic salt may be used, or two or more kinds thereof may be used in combination.

The molecular weight of the compound (B) may be 50 or more or 100 or more, and may be 400 or less or 300 or less. The molecular weight of the compound (B) that is the inorganic salt may be 50 or more or 100 or more, and may be 300 or less or 200 or less. The molecular weight of the compound (B) that is the organic acid may be 50 or more or 100 or more, and may be 200 or less or 150 or less. The molecular weight of the compound (B) that is the organic salt may be 50 or more or 100 or more, and may be 400 or less or 200 or less.

From the viewpoint of easily adjusting the electrical conductivity of the storage solution, the compound (B) is preferably citric acid, a citrate, or sodium chloride. Since DNase is activated by a magnesium ion, when the compound (B) is citric acid or a citrate, the decomposition of cfDNA in the cell-containing solution can be effectively suppressed. When the compound (B) is citric acid or a citrate, an anticoagulant effect can also be exhibited when the cell-containing solution is blood.

The content of the compound (B) in the storage solution is not particularly limited as long as the electrical conductivity of the storage solution falls within the above range.

The content of the compound (B) in the storage solution is preferably 0.1 w/v% or more, more preferably 2 w/v% or more, and further preferably 3 w/v% or more, and is preferably 30 w/v% or less, more preferably 20 w/v% or less, and further preferably 10 w/v% or less. When the content of the compound (B) is the lower limit or more and the upper limit or less, the electrical conductivity of the storage solution can be easily adjusted to a suitable range.

The storage solution preferably contains a cell-membrane-permeable compound (C) having a molecular weight of 100 or less and being not frozen at 0°C or a cell-membrane-impermeable compound (D) having a molecular weight of 300 or more. The storage solution more preferably contains a cell-membrane-permeable compound (C) having a molecular weight of 100 or less and being not frozen at 0°C and a cell-membrane-impermeable compound (D) having a molecular weight of 300 or more. In this case, the effect of the present invention can be more effectively exhibited, and the storage stability of cfDNA contained in the cell-containing solution can be more enhanced.

Whether a certain compound is a cell-membrane-permeable compound or a cell-membrane-impermeable compound is usually determined by the molecular weight, charge, polarity, and the like of the compound. A compound having no charge is normally classified as a cell-membrane-permeable compound because it is permeable to the cell membrane by passive diffusion. For example, a compound having a charge is normally classified as a cell-membrane-impermeable compound because it is impermeable to the cell membrane by passive diffusion.

<Cell-membrane-permeable compound (C) having molecular weight of 100 or less and being not frozen at 0°C>

The storage solution preferably contains a cell-membrane-permeable compound having a molecular weight of 100 or less and being not frozen at 0°C (in the present specification, referred to as "cell-membrane-permeable compound (C)"). The cell-membrane-permeable compound (C) is a compound that is permeable to the cell membrane. The cell-membrane-permeable compound (C) is different from the formaldehyde donor compound (A) and is different from the compound (B). By using the cell-membrane-permeable compound (C), the inside of the cell can be favorably dehydrated, and the cell-membrane-permeable compound (C) can be favorably retained in the cell. Therefore, it is possible to effectively suppress contamination of genomic DNA in cells into the cell-containing solution due to destruction or death of cells during storage. The cell-membrane-permeable compound (C) is also known as a cryoprotectant for cells. Only one kind of the cell-membrane-permeable compound (C) may be used, or two or more kinds thereof may be used in combination.

The molecular weight of the cell-membrane-permeable compound (C) is 100 or less. The molecular weight of the cell-membrane-permeable compound (C) may be 30 or more, 50 or more, or 95 or less.

The cell-membrane-permeable compound (C) is preferably a liquid at 0°C and preferably a liquid at 25°C.

Examples of the cell-membrane-permeable compound (C) include a polyhydric alcohol, dimethyl sulfoxide, and acetamide. Examples of the polyhydric alcohol include ethylene glycol, propylene glycol, glycerin, 1,3-propanediol, and butylene glycol.

The cell-membrane-permeable compound (C) is preferably ethylene glycol, propylene glycol, glycerin, dimethyl sulfoxide, acetamide, 1,3-propanediol, or butylene glycol, and more preferably ethylene glycol, propylene glycol, glycerin, or dimethyl sulfoxide. The cell-membrane-permeable compound (C) is preferably a polyhydric alcohol or dimethyl sulfoxide and more preferably a polyhydric alcohol. In this case, the effect of the present invention can be more effectively exhibited, and the storage stability of cfDNA can also be more enhanced.

The content of the cell-membrane-permeable compound (C) in the storage solution is not particularly limited. The content of the cell-membrane-permeable compound (C) in the storage solution can be appropriately changed, for example, so that the content of the cell-membrane-permeable compound (C) satisfies the range described below in the mixed solution R described below. The content of the cell-membrane-permeable compound (C) in the storage solution can be appropriately changed according to the mixing ratio of the cell-containing solution and the storage solution, and the like.

The content of the cell-membrane-permeable compound (C) in the storage solution is preferably 2 vol% or more, more preferably 10 vol% or more, and further preferably 20 vol% or more, and preferably 50 vol% or less, more preferably 40 vol% or less, further preferably 35 vol% or less, and particularly preferably 30 vol% or less. When the content of the cell-membrane-permeable compound (C) is the lower limit or more and the upper limit or less, the content of the cell-membrane-permeable compound (C) in the mixed solution (mixed solution R) of the cell-containing solution and the storage solution is easily adjusted to a range described below, and as a result, the effect of the present invention can be more effectively exhibited.

<Cell-membrane-impermeable compound (D) having molecular weight of 300 or more>

The storage solution preferably contains a cell-membrane-impermeable compound having a molecular weight of 300 or more (in the present specification, referred to as "cell-membrane-impermeable compound (D)"). The cell-membrane-impermeable compound (D) is different from the formaldehyde donor compound (A) and is different from the compound (B). By using the cell-membrane-impermeable compound (D), aggregation of cells can be effectively suppressed, and the protective effect on the cell membrane can be enhanced. Therefore, it is possible to effectively suppress contamination of genomic DNA in cells into the cell-containing solution due to destruction or death of cells during storage. Only one kind of the cell-membrane-impermeable compound (D) may be used, or two or more kinds thereof may be used in combination.

The molecular weight of the cell-membrane-impermeable compound (D) is 300 or more. The molecular weight of the cell-membrane-impermeable compound (D) means a molecular weight that can be calculated from a structural formula of the cell-membrane-impermeable compound (D) when the structural formula thereof can be specified, and means a weight average molecular weight when the structural formula thereof cannot be specified.

The molecular weight (weight average molecular weight) of the cell-membrane-impermeable compound (D) is preferably 1000 or more, more preferably 10,000 or more, and further preferably 100,000 or more, and is preferably 4,000,000 or less, and more preferably 1,000,000 or less. When the content (weight average molecular weight) of the cell-membrane-impermeable compound (D) is the lower limit or more and the upper limit or less, the effect of the present invention can be more effectively exhibited.

The weight average molecular weight means a weight average molecular weight in terms of polystyrene measured by gel permeation chromatography (GPC).

Examples of the cell-membrane-impermeable compound (D) include polyvinylpyrrolidone, polyethylene glycol, polyvinyl alcohol, a polysaccharide, a polysaccharide derivative, sugar alcohol, and ficoll. Examples of the polysaccharide include cellulose and dextran. Examples of the polysaccharide derivative include hydroxypropyl cellulose.

From the viewpoint of more effectively exhibiting the effect of the present invention, the cell-membrane-impermeable compound (D) is preferably polyvinylpyrrolidone, polyethylene glycol, polyvinyl alcohol, a polysaccharide, a polysaccharide derivative, sugar alcohol, or ficoll. From the viewpoint of more effectively exhibiting the effect of the present invention, the cell-membrane-impermeable compound (D) is more preferably polyvinylpyrrolidone, polyethylene glycol, polyvinyl alcohol, dextran, or hydroxypropyl cellulose, and further preferably polyvinylpyrrolidone, polyethylene glycol, or polyvinyl alcohol.

The content of the cell-membrane-impermeable compound (D) in the storage solution is not particularly limited. The content of the cell-membrane-impermeable compound (D) in the storage solution can be appropriately changed, for example, so that the content of the cell-membrane-impermeable compound (D) satisfies the range described below in the mixed solution S described below. The content of the cell-membrane-impermeable compound (D) in the storage solution can be appropriately changed according to the mixing ratio of the cell-containing solution and the storage solution, and the like.

The content of the cell-membrane-impermeable compound (D) in the storage solution is preferably 1 $\mu$mol/L or more and more preferably 5 $\mu$mol/L or more, and is preferably 100 $\mu$mol/L or less and more preferably 50 $\mu$mol/L or less. When the content of the cell-membrane-impermeable compound (D) is the lower limit or more and the upper limit or less, the content of the cell-membrane-impermeable compound (D) in the mixed solution (mixed solution S) of the cell-containing solution and the storage solution is easily adjusted to a range described below, and as a result, the effect of the present invention can be more effectively exhibited.

<Compound (E) that is different from both citric acid and citrate and has chelating action>

The storage solution preferably contains a compound that is different from both citric acid and a citrate and has a chelating action (in the present specification, referred to as "compound (E) having a chelating action"). The compound (E) having a chelating action is different from both citric acid and a citrate. The compound (E) having a chelating action is different from the formaldehyde donor compound (A), is different from the compound (B), is different from the cell-membrane-permeable compound (C), and is different from the cell-membrane-impermeable compound (D). Since DNase is activated by a magnesium ion, by using the compound (E) having a chelating action, the decomposition of cfDNA in the cell-containing solution can be effectively suppressed. When the cell-containing solution is blood, the storage solution preferably contains an anticoagulant (anticoagulant different from both citric acid and a citrate) as the compound (E) having a chelating action. In this case, in addition to effectively suppressing the decomposition of cfDNA, coagulation of blood during storage can be effectively suppressed. Only one kind of the compound (E) having a chelating action may be used, or two or more kinds thereof may be used in combination.

Examples of the compound (E) having a chelating action include ethylenediaminetetraacetic acid (EDTA) and glycol ether diaminetetraacetic acid (EGTA).

From the viewpoint of more effectively suppressing the decomposition of cfDNA and viewpoint of more effectively suppressing coagulation of blood during storage when the cell-containing solution is blood, the compound (E) having a chelating action preferably contains EDTA and is preferably EDTA.

The content of the compound (E) having a chelating action in the storage solution can be appropriately changed according to the mixing ratio of the cell-containing solution and the storage solution, the type of the compound (E) having a chelating action, and the like.

<Monosaccharide or disaccharide>

The storage solution preferably contains a monosaccharide or disaccharide. The monosaccharide or disaccharide is different from the formaldehyde donor compound (A), is different from the compound (B), is different from the cell-membrane-permeable compound (C), is different from the cell-membrane-impermeable compound (D), and is different from the compound (E) having a chelating action. By using the monosaccharide or disaccharide, the osmotic pressure of the solution obtained by mixing the cell-containing solution and the storage solution can be effectively increased, and the effect of the present invention can be more effectively exhibited. The storage solution may contain a monosaccharide, a disaccharide, or a monosaccharide and a disaccharide.

Examples of the monosaccharide include glucose, fructose, and galactose. Only one kind of the monosaccharide may be used, or two or more kinds thereof may be used in combination.

Examples of the disaccharide include sucrose, lactose, maltose, and trehalose. Only one kind of the disaccharide may be used, or two or more kinds thereof may be used in combination.

From the viewpoint of more effectively exhibiting the effect of the present invention, the monosaccharide or disaccharide is preferably glucose or sucrose, and more preferably glucose.

The content of the monosaccharide or disaccharide in the storage solution is not particularly limited. The content of the monosaccharide or disaccharide in the storage solution can be appropriately changed, for example, so that the osmotic pressure of the storage solution or the mixed solution Q described below satisfies the range described below.

<Other components>

The storage solution may contain components other than the components described above. Examples of the other components include amino acid, water, and a pH adjuster. Only one kind of the other components may be used, or two or more kinds thereof may be used in combination.

The storage solution preferably contains water. The content of the water in 100 wt% of the storage solution is preferably 30 wt% or more and more preferably 40 wt% or more, and is preferably 80 wt% or less, more preferably 70 wt% or more, and further preferably 60 wt% or less.

<Other details of storage solution>

The osmotic pressure of the storage solution is preferably 300 mOsm/L or more and more preferably 600 mOsm/L or more, and is preferably 4000 mOsm/L or less and more preferably 3000 mOsm/L or less. When the osmotic pressure of the storage solution is the lower limit or more and the upper limit or less, the effect of the present invention can be more effectively exhibited.

The osmotic pressure of the storage solution can be measured using an osmotic pressure meter (for example, "Osmometer 3250" manufactured by Advanced Instruments).

The pH of the storage solution is preferably 5.0 or more and more preferably 6.0 or more, and is preferably 8.0 or less and more preferably 7.5 or less. When the pH is the lower limit or more and the upper limit or less, the effect of the present invention can be more effectively exhibited.

(Storage container for cell-containing solution)

A storage container for a cell-containing solution according to the present invention (hereinafter, abbreviated as "storage container") is a storage container in which a predetermined amount of a cell-containing solution is collected. The storage container according to the present invention is a container for storing a cell-containing solution. The storage container according to the present invention includes a container body and a storage solution stored in the container body. In the storage container according to the present invention, the storage solution is the above-described storage solution. The storage container according to the present invention is a container for collecting a predetermined amount of a cell-containing solution and storing a mixed solution of the cell-containing solution and the storage solution. The storage container according to the present invention is used for storing a mixed solution of the collected cell-containing solution and the storage solution. The storage container according to the present invention is preferably used for storing the mixed solution under refrigeration.

The storage container preferably includes the following configuration P.

Configuration P: Sodium hypochlorite (7.4 g) is mixed with 1 L of physiological saline to obtain a solution Pa. When the solution Pa is collected in the storage container for a cell-containing solution in an amount equivalent to a predetermined amount of the cell-containing solution collected in the storage container for a cell-containing solution, and the solution Pa and the storage solution are mixed to obtain a mixed solution P, a content of formaldehyde in the mixed solution P is 100 mg/L or more and 400 mg/L or less.

The solution Pa is a solution simulating the cell-containing solution. When formaldehyde is released from the formaldehyde donor compound (A), the mixed solution P contains formaldehyde.

Specifically, the mixed solution P is prepared as follows.

The solution Pa is collected in the storage container in an amount equivalent to a predetermined amount of the cell-containing solution collected in the storage container. For example, in a storage container in which 5 mL of blood is collected, 5 mL of the solution Pa is collected in the storage container. The collected predetermined amount of the solution Pa and the storage solution are mixed by inversion mixing to obtain a mixed solution P.

The content of formaldehyde in the mixed solution P is preferably 100 mg/L or more and more preferably 150 mg/L or more, and is preferably 400 mg/L or less, more preferably 300 mg/L or less, further preferably 200 mg/L or less, and particularly preferably 190 mg/L or less. When the content of the formaldehyde in the mixed solution P is the lower limit or more and the upper limit or less, the concentration of formaldehyde in the mixed solution of the cell-containing solution and the storage solution can be appropriately controlled. Therefore, the stability of cells can be enhanced, the effect of the present invention is more effectively exhibited, and the decomposition of cfDNA can be suppressed. When the content of formaldehyde in the mixed solution P is more than 400 mg/L, as compared with the case of 400 mg/L or less, cfDNAs are easily crosslinked with each other or cfDNA and the membrane protein are easily crosslinked with each other, and the storage stability of cfDNA may be deteriorated.

The content of the formaldehyde in the mixed solution P is determined by an MBTH colorimetric method.

The storage container preferably includes the following configuration Q.

Configuration Q: when physiological saline is collected in the storage container for a cell-containing solution in an amount equivalent to a predetermined amount of the cell-containing solution collected in the storage container for a cell-containing solution, and the physiological saline and the storage solution are mixed to obtain a mixed solution Q, an osmotic pressure of the mixed solution Q is 300 mOsm/L or more and 1100 mOsm/L or less.

Specifically, the mixed solution Q is prepared as follows.

The physiological saline is collected in the storage container in an amount equivalent to a predetermined amount of the cell-containing solution collected in the storage container. For example, in a storage container in which 5 mL of blood is collected, 5 mL of the physiological saline is collected in the storage container. The collected predetermined amount of the physiological saline and the storage solution are mixed by inversion mixing to obtain a mixed solution Q.

The osmotic pressure of the mixed solution Q is preferably 300 mOsm/L or more and more preferably 350 mOsm/L or more, and is preferably 1100 mOsm/L or less and more preferably 1000 mOsm/L or less. When the osmotic pressure of the mixed solution Q is the lower limit or more and the upper limit or less, the osmotic pressure in the mixed solution of the cell-containing solution and the storage solution can be improved. Therefore, the effect of the present invention can be more effectively exhibited.

The osmotic pressure of the mixed solution Q can be measured using an osmotic pressure meter (for example, "Osmometer 3250" manufactured by Advanced Instruments).

When the storage solution contains the cell-membrane-permeable compound (C), the storage container preferably includes the following configuration R.

Configuration R: when the predetermined amount of the cell-containing solution is collected in the storage container for a cell-containing solution, and the cell-containing solution and the storage solution are mixed to obtain a mixed solution R, a content of the cell-membrane-permeable compound (C) in the mixed solution R is 1 vol% or more and 5 vol% or less.

Specifically, the mixed solution R is prepared as follows.

A predetermined amount of the cell-containing solution to be collected in the storage container is collected in the storage container. For example, in a storage container in which 5 mL of blood is collected, 5 mL of blood is collected in the storage container. The collected predetermined amount of the cell-containing solution and the storage solution are mixed by inversion mixing to obtain a mixed solution R.

The content of the cell-membrane-permeable compound (C) in the mixed solution R is preferably 1 vol% or more and more preferably 2 vol% or more, and is preferably 5 vol% or less and more preferably 4 vol% or less. That is, the content of the cell-membrane-permeable compound (C) in the mixed solution R is preferably 1 vol% or more and more preferably 2 vol% or more, and is preferably 5 vol% or less and more preferably 4 vol% or less. When the content of the cell-membrane-permeable compound (C) in the mixed solution R is the lower limit or more and the upper limit or less, the inside of the cell can be more favorably dehydrated, and the cell-membrane-permeable compound (C) can be more favorably retained in the cell. Therefore, it is possible to more effectively suppress contamination of genomic DNA in cells into the cell-containing solution due to destruction or death of cells during storage.

When the storage solution contains the cell-membrane-impermeable compound (D), the storage container preferably includes the following configuration S.

Configuration S: when the predetermined amount of the cell-containing solution is collected in the storage container for a cell-containing solution, and the cell-containing solution and the storage solution are mixed to obtain a mixed solution S, a content of the cell-membrane-impermeable compound (D) in the mixed solution S is 0.5 μmol/L or

more and 5 μmol/L or less.

Specifically, the mixed solution S is prepared as follows.

A predetermined amount of the cell-containing solution to be collected in the storage container is collected in the storage container. For example, in a storage container in which 5 mL of blood is collected, 5 mL of blood is collected in the storage container. The collected predetermined amount of the cell-containing solution and the storage solution are mixed by inversion mixing to obtain a mixed solution S. The mixed solution R and the mixed solution S are the same solution.

The content of the cell-membrane-impermeable compound (D) in the mixed solution S is preferably 0.5 μmol/L or more and more preferably 1 μmol/L or more, and is preferably 5 μmol/L or less and more preferably 4 μmol/L or less. When the content of the cell-membrane-impermeable compound (D) in the mixed solution S is the lower limit or more and the upper limit or less, aggregation of cells can be more effectively suppressed, and the protective effect on the cell membrane can be further enhanced. Therefore, it is possible to more effectively suppress contamination of genomic DNA in cells into the cell-containing solution due to destruction or death of cells during storage.

When the storage solution contains the compound (E) having a chelating action, the storage container preferably includes the following configuration T.

Configuration T: when the predetermined amount of the cell-containing solution is collected in the storage container for a cell-containing solution, and the cell-containing solution and the storage solution are mixed to obtain a mixed solution T, a content of the compound (E) having a chelating action in the mixed solution T is 0.03 w/v% or more and 0.5 w/v% or less.

Specifically, the mixed solution T is prepared as follows.

A predetermined amount of the cell-containing solution to be collected in the storage container is collected in the storage container. For example, in a storage container in which 5 mL of blood is collected, 5 mL of blood is collected in the storage container. The collected predetermined amount of the cell-containing solution and the storage solution are mixed by inversion mixing to obtain a mixed solution T. The mixed solution R, the mixed solution S, and the mixed solution T are the same solution.

The content of the compound (E) having a chelating action in the mixed solution T is preferably 0.04 w/v% or more and more preferably 0.06 w/v% or more, and is preferably 0.40 w/v% or less and more preferably 0.30 w/v% or less. When the content of the compound (E) having a chelating action in the mixed solution T is the lower limit or more and the upper limit or less, the decomposition of cfDNA can be more effectively suppressed. When the content of the compound (E) having a chelating action is the lower limit or more and the upper limit or less, coagulation of blood during storage can be more effectively suppressed when the cell-containing solution is blood.

<Container body>

The shape of the container body is not particularly limited. The shape of the container body is preferably a bottomed container, and more preferably a bottomed tubular container.

The material for the container body is not particularly limited. Examples of the material for the container body include thermoplastic resins such as polyethylene, polypropylene, polystyrene, polyethylene terephthalate, polymethyl methacrylate, and polyacrylonitrile; thermosetting resins such as an unsaturated polyester resin, an epoxy resin, and epoxy-acrylate resin; modified natural resins such as cellulose acetate, cellulose propionate, ethyl cellulose, and ethylchitin; silicate glasses such as soda-lime glass, phosphosilicate glass, and borosilicate glass, and glasses such as quartz glass. Only one kind of the material of the container body may be used, or two or more kinds thereof may be used in combination.

<Plug>

The storage container preferably includes a plug. As the plug, a conventionally known plug can be used. The plug is preferably a plug that is formed of a material or shape that can be airtightly and liquid-tightly attached to an opening of the container body. When the cell-containing solution is blood, the plug is preferably configured such that a blood sampling needle can be inserted.

Examples of the plug include a plug having a shape fitted to the opening of the container body and a sheet-shaped seal plug.

The plug may be a plug including a plug body such as a rubber plug and a cap member made of plastic or the like. In this case, it is possible to suppress the risk that the blood comes into contact with the human body when the plug is pulled out from the opening of the container body after the blood is collected.

Examples of the material for the plug (or the plug body) include synthetic resins, elastomers, rubbers, and metal foils. Examples of the rubbers include butyl rubber and halogenated butyl rubber. Examples of the metal foils include an aluminum foil. From the viewpoint of enhancing the sealing property, the material for the plug is preferably butyl rubber. The plug is preferably a butyl rubber plug.

(Other details of storage container and storage solution)

A predetermined amount of the cell-containing solution is collected in the storage container. The storage container is a container for collecting and storing a predetermined amount of the cell-containing solution. The storage container is a container for storing the cell-containing solution and the storage solution in a mixed state. The predetermined amount of the cell-containing solution collected in the storage container is appropriately changed according to the size of the storage container, and the like. The predetermined amount of the cell-containing solution collected in the storage container is, for example, 1 mL, 5 mL, 10 mL, or 20 mL. The predetermined amount of the cell-containing solution collected in the storage container may be 0.5 mL or more, 1 mL or more, or 5 mL or more, and may be 25 mL or less, 20 mL or less, or 15 mL or less.

The storage container is preferably a container for storing the cell-containing solution in a liquid state, and is preferably a container for storing the cell-containing solution without freezing. The storage container is preferably a container for storing the cell-containing solution at 0°C or higher and more preferably a container for storing the cell-containing solution at 1°C or higher, and is preferably a container for storing the cell-containing solution at 40°C or lower, more preferably a container for storing the cell-containing solution at 25°C or lower, and further preferably a container for storing the cell-containing solution at 10°C or lower.

The storage solution is used for storing the cell-containing solution preferably at 0°C or higher and more preferably at 1°C or higher, and is used for storing the cell-containing solution preferably at 40°C or lower, more preferably at 25°C or lower, and further preferably at 10°C or lower.

The amount of the storage solution stored in the container body with respect to 1 mL of the collected cell-containing solution is preferably 0.05 mL or more and more preferably 0.1 mL or more, and is preferably 1 mL or less and more preferably 0.5 mL or less. When the amount of the storage solution is the lower limit or more and the upper limit or less, the effect of the present invention can be more effectively exhibited without excessively diluting the cell-containing solution.

The storage solution is mixed and used in an amount of preferably 0.05 mL or more and more preferably 0.1 mL or more, and is mixed and used in an amount of preferably 1 mL or less and more preferably 0.5 mL or less, with respect to 1 mL of the collected cell-containing solution. In this case, the effect of the present invention can be more effectively exhibited without excessively diluting the cell-containing solution.

The internal pressure of the storage container is not particularly limited. The internal pressure of the storage container is preferably reduced. When the storage container is a depressurization container, a predetermined amount of the cell-containing solution can be easily collected in the storage container. The storage container can also be used as a vacuum blood sampling tube sealed by the plug after the inside is evacuated. In the case of the vacuum blood sampling tube, it is possible to easily sample a certain amount of blood regardless of the skills of the blood sampling operator.

From the viewpoint of preventing bacterial infection, the inside of the storage container is preferably sterilized in compliance with the criteria of ISO or JIS.

Hereinafter, the present invention will be described in more detail with reference to Examples. The present invention is not limited only to the following Examples.

As materials for the storage solution, the following were prepared.

(Formaldehyde donor compound (A))

1-Hydroxymethyl-5,5-dimethylhydantoin
DMDM hydantoin
Imidazolidinyl urea

(Compound (B) that is inorganic salt, organic acid, or organic salt)

3Na citrate·2H$_2$O (containing 3Na citrate as an organic salt)
Citric acid·H$_2$O (containing citric acid as an organic acid)
Sodium chloride

(Cell-membrane-permeable compound (C))

Propylene glycol
Dimethyl sulfoxide

(Cell-membrane-impermeable compound (D))

Polyethylene glycol (weight average molecular weight: 500,000)
Dextran (weight average molecular weight: 200,000)

(Compound (E) having chelating action)

EDTA (anticoagulant)

(Others)

Glucose
Glycine
Water

(Examples 1 to 8 and Comparative Examples 2 and 3)

Preparation of storage solution:

Blending components shown in Tables 1 to 3 were blended with water in blending amounts shown in Tables 1 to 3 to obtain a storage solution. The blending amounts of the organic acid and the organic salt of the compound (B) were shown as the blending amounts in the state of a hydrate.

Preparation of storage container:

As the container body, a polyethylene terephthalate bottomed tube (PET bottomed tube) having a length of 100 mm and an opening with an inner diameter of 14 mm was prepared. In the PET bottomed tube, 0.5 mL of the obtained storage solution was stored. The inside of the storage container was depressurized to 50 kPa and sealed with a butyl rubber plug. In this way, a storage container for collecting and storing 5 mL of blood was prepared.

(Comparative Example 1)

Preparation of storage solution:

Blending components shown in Table 3 were blended with water in blending amounts shown in Table 3 to obtain a storage solution.

Preparation of storage container:

As the container body, a polyethylene terephthalate bottomed tube (PET bottomed tube) having a length of 100 mm and an opening with an inner diameter of 14 mm was prepared. In the PET bottomed tube, 0.05 mL of the obtained storage solution was stored. The inside of the storage container was depressurized to 50 kPa and sealed with a butyl rubber plug. In this way, a storage container for collecting and storing 5 mL of blood was prepared.

(Evaluation)

(1) Electrical conductivity of storage solution

The electrode was immersed in 15 mL of the obtained storage solution, and the electrical conductivity was measured. The electrical conductivity of the storage solution was measured at 25.0°C using a cell having a cell constant of $0.949 \times 0.1 cm^{-1}$ and an electrical conductivity meter ("DS-71" manufactured by HORIBA Scientific).

(2) Content of formaldehyde in mixed solution P

Sodium hypochlorite (7.4 g) was mixed with 1 L of physiological saline to prepare a solution Pa. In the obtained storage container, 5 mL of the solution Pa was collected, and the solution Pa and the storage solution were mixed by inversion mixing to obtain a mixed solution P. The content (mg/L) of formaldehyde in the obtained mixed solution P was determined by an MBTH colorimetric method.

(3) Osmotic pressure of mixed solution Q

In the obtained storage container, 5 mL of the physiological saline was collected, and the physiological saline and the storage solution were mixed by inversion mixing to obtain a mixed solution Q. The osmotic pressure of the obtained mixed solution Q was measured using an osmotic pressure meter ("Osmometer 3250" manufactured by Advanced Instruments).

(4) Content of cell-membrane-permeable compound (C) in mixed solution R

In the obtained storage container, 5 mL of blood was collected, and the blood and the storage solution were mixed by inversion mixing to obtain a mixed solution R. The content (vol%) of the cell-membrane-permeable compound (C) contained in the obtained mixed solution R was determined.

(5) Content of cell-membrane-impermeable compound (D) in mixed solution S

In the obtained storage container, 5 mL of blood was collected, and the blood and the storage solution were mixed by inversion mixing to obtain a mixed solution S. The content ($\mu$mol/L) of the cell-membrane-impermeable compound (D) contained in the obtained mixed solution S was determined.

(6) Storage stability (4°C)

(6-1) Hemolysis

In the obtained storage container, 5 mL of blood was collected, and the blood and the storage solution were mixed by inversion mixing. The storage container in which the mixed solution had been stored was stored in an environment of 4°C. After 7 days from the storage, the storage container in which the mixed solution had been stored was centrifuged to separate the mixed solution into a layer containing plasma (upper side) and a layer containing a cell component (lower side). The layer containing plasma was visually confirmed, and whether or not hemolysis occurred in the layer containing plasma was confirmed.

<Criteria for hemolysis>

O: No hemolysis occurs in the layer containing plasma.
X: Hemolysis occurs in the layer containing plasma.

(6-2) DNA concentration in plasma (leakage of genomic DNA to outside of cells)

After the test of "(6-1) Hemolysis" described above was performed, the layer containing plasma was collected from the storage container. DNA contained in the collected plasma was purified using cfDNA purification kit ("QIAamp Circulating Nucleic Acid Kit" manufactured by QIAGEN). The DNA purification operation was performed on the day when the plasma was collected from the storage container.

The DNA concentration in the extract after purification was measured using Qubit dsDNA HS Assay kit (Invitrogen). The DNA concentration (content of DNA contained per 1 mL of plasma) was calculated according to the following formula.

$$\text{DNA concentration (ng/1 mL plasma)} = [A] \times [B]/[C]$$

[A]: Measured value (ng/mL) of DNA concentration in extract after purification
[B] : Total dose (mL) of extract after purification (mL)
[C]: Dose (mL) of plasma used for DNA purification

A lower DNA concentration (ng/1 mL plasma) means that leakage of genomic DNA to the outside of cells is suppressed.
Compositions and results are shown in Tables 1 to 3 below.

[Table 1]

| | | | Example 1 | Example 2 | Example 3 | Example 4 |
|---|---|---|---|---|---|---|
| Storage solution (components other than water) | Formaldehyde donor compound (A) | 1-Hydroxymethyl-5,5-dimethylhydantoin | 1.1 w/v% | 1.1 w/v% | 1.1 w/v% | 1.1 w/v% |
| | | DMDM hydantoin | - | - | - | - |
| | | Imidazolidinyl urea | - | - | - | - |
| | Compound (E) that is inorganic salt, organic acid, or organic salt | 3Na citrate·2H$_2$O | 3.6 w/v% | - | - | 6.0 w/v% |
| | | Citric acid·H$_2$O | 0.13 w/v% | - | - | 0.13 w/v% |
| | | Sodium chloride | - | 0.98 w/v% | 0.66 w/v% | - |
| | Cell-membrane-permeable compound (C) | Propylene glycol | 33.0 vol% | 33.0 vol% | 33.0 vol% | 33.0 vol% |
| | | Dimethyl sulfoxide | - | - | - | - |
| | Cell-membrane-impermeable compound (D) | Polyethylene glycol (MW: 500,000) | 11 μmol/L | 11 μmol/L | 11 μmol/L | 11 μmol/L |
| | | Dextran (MW: 200,000) | - | - | - | - |
| | Compound (E) having chelating action | EDTA | 2.25 w/v% | 2.25 w/v% | 2.25 w/v% | 2.25 w/v% |
| | Others | Glucose | 20 w/v% | 20 w/v% | 20 w/v% | 20 w/v% |
| | | Glycine | - | - | - | - |
| Electrical conductivity (ms/cm) of storage solution | | | 4.15 | 4.98 | 3.71 | 4.66 |
| Mixed solution P | Content (mg/L) of formaldehyde | | 180 | 180 | 180 | 180 |
| Mixed solution Q | Osmotic pressure (mOsm/L) | | 935 | 920 | 902 | 957 |
| Mixed solution R | Content (vol%) of cell-membrane-permeable compound (C) | | 3.0 | 3.0 | 3.0 | 3.0 |
| Mixed solution S | Content (μmol/L) of cell-membrane-impermeable compound (D) | | 1.0 | 1.0 | 1.0 | 1.0 |

(continued)

|  |  | Example 1 | Example 2 | Example 3 | Example 4 |
|---|---|---|---|---|---|
| Storage stability (4°C) | Hemolysis | ○ | ○ | ○ | ○ |
| | DNA concentration in plasma (ng/1 mL plasma) (leakage of genomic DNA to outside of cells) | 11 | 8 | 9 | 8 |

[Table 2]

| | | | Example 5 | Example 6 | Example 7 | Example 8 |
|---|---|---|---|---|---|---|
| Storage solution (components other than water) | Formaldehyde donor compound (A) | 1-Hydroxymethyl-5,5-dimethylhydantoin | 1.1 w/v% | - | 1.1 w/v% | 1.1 w/v% |
| | | DMDM hydantoin | - | 1.1 w/v% | - | - |
| | | Imidazolidinyl urea | - | - | - | - |
| | Compound (E) that is inorganic salt, organic acid, or organic salt | 3Na citrate·2H$_2$O | - | - | - | - |
| | | Citric acid·H$_2$O | - | - | - | - |
| | | Sodium chloride | 4.8 w/v% | 0.66 w/v% | 0.66 w/v% | 0.66 w/v% |
| | Cell-membrane-permeable compound (C) | Propylene glycol | 33.0 vol% | 33.0 vol% | - | 33.0 vol% |
| | | Dimethyl sulfoxide | - | - | 33.0 vol% | - |
| | Cell-membrane-impermeable compound (D) | Polyethylene glycol (MW: 500,000) | 11 μmol/L | 11 μmol/L | 11 μmol/L | - |
| | | Dextran (MW: 200,000) | - | - | - | 11 μmol/L |
| | Compound (E) having chelating action | EDTA | 2.25 w/v% | 2.25 w/v% | 2.25 w/v% | 2.25 w/v% |
| | Others | Glucose | 20 w/v% | 20 w/v% | 20 w/v% | 20 w/v% |
| | | Glycine | - | - | - | - |
| Electrical conductivity (ms/cm) of storage solution | | | 18.22 | 3.71 | 3.35 | 4.1 |
| Mixed solution P | Content (mg/L) of formaldehyde | | 180 | 170 | 180 | 180 |
| Mixed solution Q | Osmotic pressure (mOsm/L) | | 1080 | 902 | 920 | 900 |
| Mixed solution R | Content (vol%) of cell-membrane-permeable compound (C) | | 3.0 | 3.0 | 3.0 | 3.0 |
| Mixed solution S | Content (μmol/L) of cell-membrane-impermeable compound (D) | | 1.0 | 1.0 | 1.0 | 1.0 |

(continued)

|  |  | Example 5 | Example 6 | Example 7 | Example 8 |
|---|---|---|---|---|---|
| Storage stability (4°C) | Hemolysis | ○ | ○ | ○ | ○ |
|  | DNA concentration in plasma (ng/1 mL plasma) (leakage of genomic DNA to outside of cells) | 6 | 8 | 8 | 11 |

[Table 3]

| | | | Comparative Example 1 | Comparative Example 2 | Comparative Example 3 |
|---|---|---|---|---|---|
| Storage solution (components other than water) | Formaldehyde donor compound (A) | 1-Hydroxymethyl-5,5-dimethylhydantoin | - | - | 1.1 w/v% |
| | | DMDM hydantoin | - | - | - |
| | | Imidazolidinyl urea | 50 w/v% | - | - |
| | Compound (E) that is inorganic salt, organic acid, or organic salt | 3Na citrate·2H$_2$O | - | - | - |
| | | Citric acid·H$_2$O | - | - | - |
| | | Sodium chloride | - | 0.91 w/v% | 0.1 w/v% |
| | Cell-membrane-permeable compound (C) | Propylene glycol | - | 33.0 vol% | 33.0 vol% |
| | | Dimethyl sulfoxide | | - | - |
| | Cell-membrane-impermeable compound (D) | Polyethylene glycol (MW: 500,000) | - | 11 μmol/L | 11 μmol/L |
| | | Dextran (MW: 200,000) | - | - | - |
| | Compound (E) having chelating action | EDTA | 10 w/v% | 2.25 w/v% | 2.25 w/v% |
| | Others | Glucose | - | 20 w/v% | 20 w/v% |
| | | Glycine | 5 w/v% | - | - |
| Electrical conductivity (ms/cm) of storage solution | | | 1.18 | 3.1 | 1.89 |
| Mixed solution P | Content (mg/L) of formaldehyde | | 360 | 0 | 180 |
| Mixed solution Q | Osmotic pressure (mOsm/L) | | 330 | 850 | 910 |
| Mixed solution R | Content (vol%) of cell-membrane-permeable compound (C) | | - | 3.0 | 3.0 |
| Mixed solution S | Content (μmol/L) of cell-membrane-impermeable compound (D) | | - | 1.0 | 1.0 |

(continued)

|  | | Comparative Example 1 | Comparative Example 2 | Comparative Example 3 |
|---|---|---|---|---|
| Storage stability (4°C) | Hemolysis | × | × | × |
| | DNA concentration in plasma (ng/1 mL plasma) (leakage of genomic DNA to outside of cells) | 24 | 110 | 16 |

**Claims**

1. A storage solution for a cell-containing solution, comprising:

   a formaldehyde donor compound (A); and
   a compound (B) that is an inorganic salt, an organic acid, or an organic salt,
   an electrical conductivity of the storage solution being 3 ms/cm or more and 20 ms/cm or less.

2. The storage solution for a cell-containing solution according to claim 1, comprising a cell-membrane-permeable compound (C) having a molecular weight of 100 or less and being not frozen at 0°C.

3. The storage solution for a cell-containing solution according to claim 2, wherein the cell-membrane-permeable compound (C) is a polyhydric alcohol.

4. The storage solution for a cell-containing solution according to claim 2, wherein the cell-membrane-permeable compound (C) is ethylene glycol, propylene glycol, glycerin, dimethyl sulfoxide, acetamide, 1,3-propanediol, or butylene glycol.

5. The storage solution for a cell-containing solution according to any one of claims 1 to 4, comprising a cell-membrane-impermeable compound (D) having a molecular weight of 300 or more.

6. The storage solution for a cell-containing solution according to claim 5, wherein the molecular weight of the cell-membrane-impermeable compound (D) is 1000 or more.

7. The storage solution for a cell-containing solution according to claim 5 or 6, wherein the cell-membrane-impermeable compound (D) is polyvinylpyrrolidone, polyethylene glycol, polyvinyl alcohol, a polysaccharide, a polysaccharide derivative, sugar alcohol, or ficoll.

8. The storage solution for a cell-containing solution according to any one of claims 1 to 7, wherein the formaldehyde donor compound (A) is DMDM hydantoin or 1-hydroxymethyl-5,5-dimethylhydantoin.

9. The storage solution for a cell-containing solution according to any one of claims 1 to 8, wherein the compound (B) is citric acid, a citrate, or sodium chloride.

10. The storage solution for a cell-containing solution according to any one of claims 1 to 9, wherein the cell-containing solution is blood.

11. A storage container for a cell-containing solution in which a predetermined amount of a cell-containing solution is collected, the storage container for a cell-containing solution, comprising:

    a container body; and
    a storage solution stored inside the container body,
    the storage solution being the storage solution for a cell-containing solution according to any one of claims 1 to 10.

12. The storage container for a cell-containing solution according to claim 11, wherein the storage container for a cell-containing solution includes the following configuration P:

the configuration P is that, when 7.4 g of sodium hypochlorite is mixed with 1 L of physiological saline to obtain a solution Pa, the solution Pa is collected in the storage container for a cell-containing solution in an amount equivalent to a predetermined amount of the cell-containing solution collected in the storage container for a cell-containing solution, and the solution Pa and the storage solution are mixed to obtain a mixed solution P, a content of formaldehyde in the mixed solution P is 100 mg/L or more and 400 mg/L or less.

13. The storage container for a cell-containing solution according to claim 11 or 12, wherein the storage container for a cell-containing solution includes the following configuration Q:
the configuration Q is that, when physiological saline is collected in the storage container for a cell-containing solution in an amount equivalent to a predetermined amount of the cell-containing solution collected in the storage container for a cell-containing solution, and the physiological saline and the storage solution are mixed to obtain a mixed solution Q, an osmotic pressure of the mixed solution Q is 300 mOsm/L or more and 1100 mOsm/L or less.

14. The storage container for a cell-containing solution according to any one of claims 11 to 13, wherein

the storage solution contains a cell-membrane-permeable compound (C) having a molecular weight of 100 or less and being not frozen at 0°C, and
the storage container for a cell-containing solution includes the following configuration R:
the configuration R is that, when the predetermined amount of the cell-containing solution is collected in the storage container for a cell-containing solution, and the cell-containing solution and the storage solution are mixed to obtain a mixed solution R, a content of the cell-membrane-permeable compound (C) in the mixed solution R is 1 vol% or more and 5 vol% or less.

15. The storage container for a cell-containing solution according to any one of claims 11 to 14, wherein

the storage solution contains a cell-membrane-impermeable compound (D) having a molecular weight of 300 or more, and
the storage container for a cell-containing solution includes the following configuration S:
the configuration S is that, when the predetermined amount of the cell-containing solution is collected in the storage container for a cell-containing solution, and the cell-containing solution and the storage solution are mixed to obtain a mixed solution S, a content of the cell-membrane-impermeable compound (D) in the mixed solution S is 0.5 $\mu$mol/L or more and 5 $\mu$mol/L or less.

## INTERNATIONAL SEARCH REPORT

| International application No. |
| --- |
| **PCT/JP2022/028168** |

**A. CLASSIFICATION OF SUBJECT MATTER**

*C12N 5/00*(2006.01)i; *C12M 1/26*(2006.01)i; *C12M 3/00*(2006.01)i; *C12N 1/04*(2006.01)i; *C12N 5/078*(2010.01)i
FI:   C12N5/00; C12N1/04; C12M1/26; C12M3/00 Z; C12N5/078

According to International Patent Classification (IPC) or to both national classification and IPC

**B. FIELDS SEARCHED**

Minimum documentation searched (classification system followed by classification symbols)

C12N5/00; C12M1/26; C12M3/00; C12N1/04; C12N5/078

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
| --- | --- | --- |
| A | WO 2019/079743 A1 (STRECK, INC.) 25 April 2019 (2019-04-25)<br>claims, paragraphs [0005], [0065]-[0068] | 1-15 |
| A | CN 112662662 A (NANCHANG ADICAN MEDICAL EXAMINATION LABORATORY CO LTD) 16 April 2021 (2021-04-16)<br>claims, paragraphs [0003]-[0015] | 1-15 |
| A | CN 103585508 A (ZHANG, Chao) 19 February 2014 (2014-02-19)<br>claims, paragraphs [0019]-[0024] | 1-15 |
| A | US 2010/0209930 A1 (STRECK, INC.) 19 August 2010 (2010-08-19)<br>claims | 1-15 |
| A | WO 2013/123030 A2 (STRECK, INC.) 22 August 2013 (2013-08-22)<br>claims, paragraph [0006] | 1-15 |
| A | WO 2018/084228 A1 (THE UNIVERSITY OF TOKYO) 11 May 2018 (2018-05-11)<br>claims | 1-15 |

☑ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **08 September 2022** | **20 September 2022** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT

| International application No. |
| --- |
| **PCT/JP2022/028168** |

**C. DOCUMENTS CONSIDERED TO BE RELEVANT**

| Category* | Citation of document, with indication, where appropriate, of the relevant passages | Relevant to claim No. |
| --- | --- | --- |
| P, A | WO 2021/177344 A1 (SEKISUI MEDICAL CO LTD) 10 September 2021 (2021-09-10) entire text | 1-15 |

Form PCT/ISA/210 (second sheet) (January 2015)

**INTERNATIONAL SEARCH REPORT**
**Information on patent family members**

International application No.

**PCT/JP2022/028168**

| Patent document cited in search report | | | Publication date (day/month/year) | Patent family member(s) | | | Publication date (day/month/year) |
|---|---|---|---|---|---|---|---|
| WO | 2019/079743 | A1 | 25 April 2019 | JP 2021-500585 A claims, paragraphs [0005], [0056]-[0059] US 2021/0371848 A1 EP 3697209 A1 | | | |
| CN | 112662662 | A | 16 April 2021 | (Family: none) | | | |
| CN | 103585508 | A | 19 February 2014 | (Family: none) | | | |
| US | 2010/0209930 | A1 | 19 August 2010 | WO 2010/096323 A1 | | | |
| WO | 2013/123030 | A2 | 22 August 2013 | US 2014/0080112 A1 EP 2814981 A | | | |
| WO | 2018/084228 | A1 | 11 May 2018 | US 2021/0169069 A1 claims EP 3564356 A1 | | | |
| WO | 2021/177344 | A1 | 10 September 2021 | (Family: none) | | | |

Form PCT/ISA/210 (patent family annex) (January 2015)

**EP 4 394 028 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2013123030 A1
- CN 107083382 A

- JP 2011111406 A